# EUROPEAN PATENT APPLICATION

(11) **EP 3 673 839 A1**
(43) Date of publication of application: **01.07.2020**
(21) Application number: 18848902.5
(22) Date of filing: 17.08.2018
(51) Int. Cl.: A61B 17/22, A61B 17/3207, A61B 17/50

(54) **INTRALUMINAL FOREIGN OBJECT CATCHING TOOL**

(30) Priority: 23.08.2017 JP 2017159973
(71) Applicant: Piolax Medical Devices, Inc., Yokohama-shi, Kanagawa 240-0023 (JP)
(72) Inventor: IRIE Toshiyuki, Mito City Ibaraki 310-0022 (JP); NANAUMI Kazutaka, Yokohama-shi Kanagawa 240-0023 (JP); ISOZAKI Shuji, Yokohama-shi Kanagawa 240-0023 (JP); PIOLAX MEDICAL DEVICES, INC., Yokohama-shi Kanagawa 240-0023 (JP)
(74) Representative: Grünecker Patent- und Rechtsanwälte PartG mbB
(86) International application number: PCT/JP2018/030465
(87) International publication number: WO 2019/039387

(57) **Abstract**

Provided is an intraluminal foreign object capturing tool with which it is possible to cause a capturing member for capturing a foreign object to expand to an appropriate diameter. The foreign object capturing tool 10, in order to capture a foreign object in a lumen, includes: a core wire 20; a capturing member 30 which is disposed movably outside the core wire 20, has a farthest tip-end which is movement-regulated at a predetermined position of the core wire 20, and expands in diameter by contracting axially; a tubular member 50 which is disposed outside the core wire 20 on a base end side of the capturing member 30, and which regulates movement of capturing member 30 on the base end side; and control members 60 which are disposed outside the core wire 20 and inside the capturing member 30, and which regulate the amount of axial contraction of the capturing member 30. As the tubular member 50 and the core wire 20 are moved relative to each other, the capturing member 30 contracts axially by a length regulated by the control member 60 and expands in diameter.

## Description

### TECHNICAL FIELD

The present invention relates to an intraluminal foreign object capturing tool for removing foreign objects, such as thrombus and stones, formed in tubular organs such as blood vessels, bile ducts, pancreatic ducts, and urinary tracts.

### BACKGROUND ART

A foreign object, such as thrombus, a gallstone, a pancreatic stone, or a ureteral stone may be produced in a tubular organ, such as a blood vessel, a bile duct, a pancreatic duct, or a urinary tract. Thus, such foreign objects are captured and removed from within the tubular organ.

For example, Patent Literature 1 describes a device for bridging a neck of an aneurysm, including: an expandable member that can be positioned within a blood vessel to form a bridge across an opening of aneurysm, the expandable member having a plurality of wires interwoven in a basket-like shape; and a pull wire coupled at least to a distal end of the expandable member, the pull wire configured to control expansion of the expandable member when an axial force component is applied to a proximal end of the wires from outside the patient. The proximal end of the expandable member is supported on a tube, the pull wire is passed through inside the tube, and the tip end of the pull wire is coupled to the distal end of the expandable member. As the pull wire is pulled toward the hand (toward the proximal end), the expansion member is compressed axially and expands (paragraphs 0069, 0070).

### CITATION LIST

### PATENT LITERATURE

Patent Literature 1: JP-A-2016-93542

### SUMMARY OF THE INVENTION

### PROBLEMS TO BE SOLVED BY THE INVENTION

When capturing a foreign object in the body, it is sometimes desirable to adjust the amount of expansion of an expansion member in order to obtain a sufficient expansive force for capturing the foreign object. In this case, with the device according to Patent Literature 1, it is necessary to pull the pull wire relative to the tube with a delicately adjusted amount of force, and it has been difficult to adjust to a desired amount of expansion. Also, if the amount by which the pull wire is pulled relative to the tube is increased so that the expansion member has a sufficient expansive force, the expansive force of the expansion member may become excessive, presenting a potential risk of damage to hollow organs or the like around the expansion member.

Accordingly, an object of the present invention is to provide an intraluminal foreign object capturing tool with which, concerning a capturing member for capturing a foreign object in a lumen, it is possible to achieve an expansion to an appropriate diameter so as to obtain a sufficient expansive force for capturing the foreign object.

### SOLUTION TO THE PROBLEMS

In order to achieve the object, the present invention provides an intraluminal foreign object capturing tool for capturing a foreign object in a lumen, the intraluminal foreign object capturing tool including: a core wire; a capturing member which is disposed movably outside the core wire, of which a farthest tip-end is movement-regulated at a predetermined position of the core wire, and which expands in diameter by contracting axially; a tubular member which is disposed outside the core wire on a base end side of the capturing member, and which regulates movement of the capturing member on the base end side; and a control member which is disposed outside the core wire and inside the capturing member, and which regulates an amount of axial contraction of the capturing member. As the tubular member and the core wire are moved relative to each other, the capturing member contracts axially by a length regulated by the control member and expands in diameter.

### EFFECTS OF THE INVENTION

According to the present invention, after being inserted into the lumen in which the foreign object exists, the tubular member and the core wire are moved relative to each other by, for example, pulling the core wire relative to the tubular member, whereby the capturing member contracts axially by a length regulated by the control member and expands in diameter. Thus, it is possible to capture the foreign object reliably. In addition, because the amount of axial contraction of the capturing member is regulated by the control member, it is possible to cause the capturing member to expand to an appropriate diameter to obtain a sufficient expansive force for capturing the foreign object.

### BRIEF DESCRIPTION OF THE DRAWINGS

Fig. 1 illustrates an embodiment of an intraluminal foreign object capturing tool according to the present invention, where (a) is a cross-sectional explanatory diagram of a capturing member when reduced in diameter, and (b) is a cross-sectional explanatory diagram of the capturing member when expanded in diameter.
Fig. 2 is a main part-enlarged cross-sectional explanatory diagram of the capturing member when reduced in diameter in the intraluminal foreign object capturing tool.
Fig. 3 is a main part-enlarged cross-sectional explanatory diagram of the capturing member when expanded in diameter in the intraluminal foreign object capturing tool.

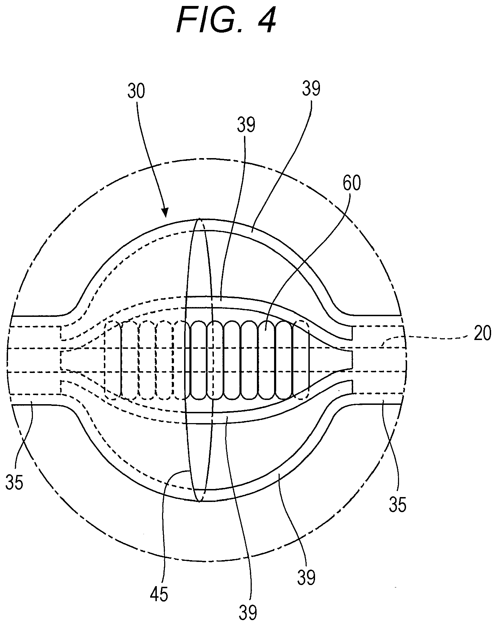

Fig. 4 is a main part-enlarged plan view of the capturing member when expanded in diameter in the intraluminal foreign object capturing tool.
Fig. 5 depicts (a) a main part-enlarged side view of a capturing portion, and (b) a main part-enlarged side view of another shape of the capturing portion, in the intraluminal foreign object capturing tool.
Fig. 6 depicts a main part-enlarged side view of yet another shape of the capturing portion in the capturing portion.
Fig. 7 illustrates use states of the foreign object capturing portion, where (a) is an explanatory diagram illustrating a first use state, (b) is an explanatory diagram illustrating a second use state, (c) is an explanatory diagram illustrating a third use state.

### DESCRIPTION OF THE EMBODIMENTS

In the following, an embodiment of an intraluminal foreign object capturing tool according to the present invention will be described with reference to the drawings.

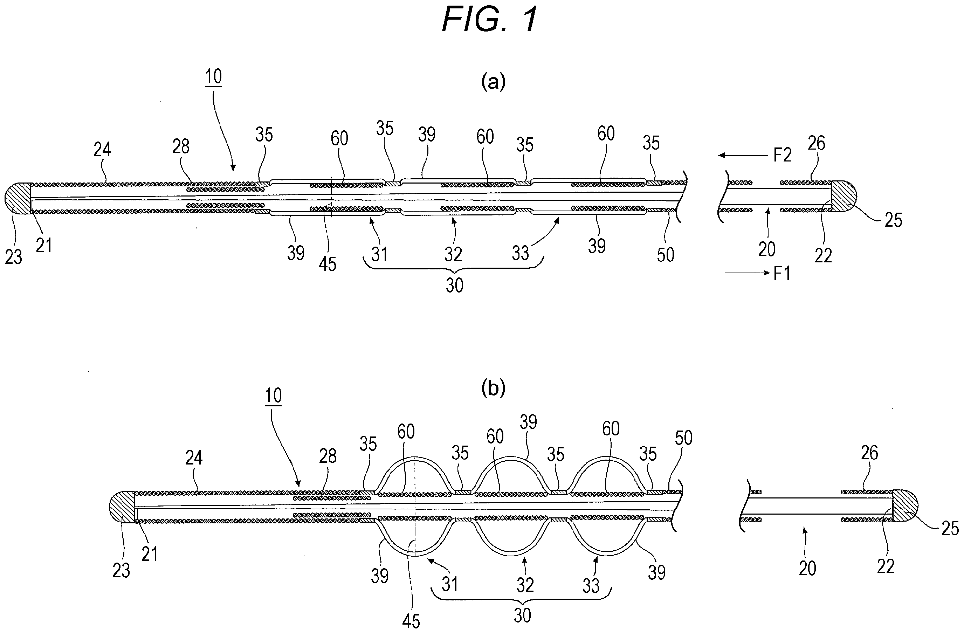

As illustrated in Fig. 1, the intraluminal foreign object capturing tool 10 (which may also be hereafter referred to as the "foreign object capturing tool 10") of the embodiment is for capturing a foreign object produced in a lumen, and includes: a core wire 20; a capturing member 30 which is movably disposed outside the core wire 20, of which a farthest tip-end is movement-regulated at a predetermined position of the core wire, and which expands in diameter by contracting axially; a tubular member 50 which is disposed outside the core wire 20 on a base end side of the capturing member 30, and which regulates movement of the capturing member 30 on the base end side; and control members 60 which are disposed outside the core wire 20 and inside the capturing member 30, and which regulate the amount of axial contraction of the capturing member 30.

The core wire 20, in the case of the embodiment, is shaped such that a base end 22 has a larger diameter, the diameter becoming gradually smaller toward an axial tip end 21. However, the shape of the core wire is not particularly limited. For example, the core wire may be shaped such that the diameter becomes smaller in a stepwise manner from the base end 22 side toward the tip end 21 side, or the core wire may have a constant diameter from the base end 22 to the tip end 21.

The tip end 21 of the core wire 20 has a hemispherical tip end-fixing portion 23 fixed thereto. A support member 24 is disposed on the tip-end side of, and externally around, the core wire 20 via the tip end-fixing portion 23. The support member 24 has the shape of a closely wound coil of wire material having a predetermined diameter, with the tip end thereof being fixed to the tip end-fixing portion 23. The support member 24 may have the shape of a coil of wire material having a wire-to-wire gap, for example, and may not be disposed externally around the core wire.

In the following description, "base end" means an end (proximal end) closer to the hand of a user using the foreign object capturing tool; "closest base end" means an end which is the closest to the user's hand; "tip end" means an end (distal end) on the side opposite to the base end; and "farthest tip end" means an end which is the farthest from the user's hand.

Meanwhile, the base end 22 of the core wire 20 also has a hemispherical base end-fixing portion 25 fixed thereto, and a holding member 26 is fixed on the base end side of, and externally around, the core wire 20 via the base end-fixing portion 25. The holding member 26 has the shape of a closely wound coil of wire material having a predetermined diameter, with the base end thereof being fixed to the base end-fixing portion 25. The holding member 26 is for making it easier for the user of the foreign object capturing tool 10 to hold the core wire 20. The holding member 26 may have the shape of a coil of wire material having a wire-to-wire gap, for example, and may not be disposed externally around the core wire.

As illustrated in Fig. 1 to Fig. 3, the capturing member 30 of the present embodiment has the shape of a tube disposed outside the core wire 20 via a predetermined gap, and is movable in the axial direction of the core wire 20. As illustrated in Fig. 1(a), in the present embodiment, the capturing member 30 includes a plurality of capturing portions 31, 32, 33 disposed in the axial direction of the core wire 20, and the control members 60 are disposed respectively inside the capturing portions 31, 32, 33. More specifically, the capturing member 30 of the present embodiment includes a plurality of annular link portions 35 disposed at predetermined intervals in the axial direction of the core wire 20, with the plurality of capturing portions 31, 32, 33 disposed between the link portions 35, and has the structure in which the plurality of capturing portions 31, 32, 33 is integrally formed axially.

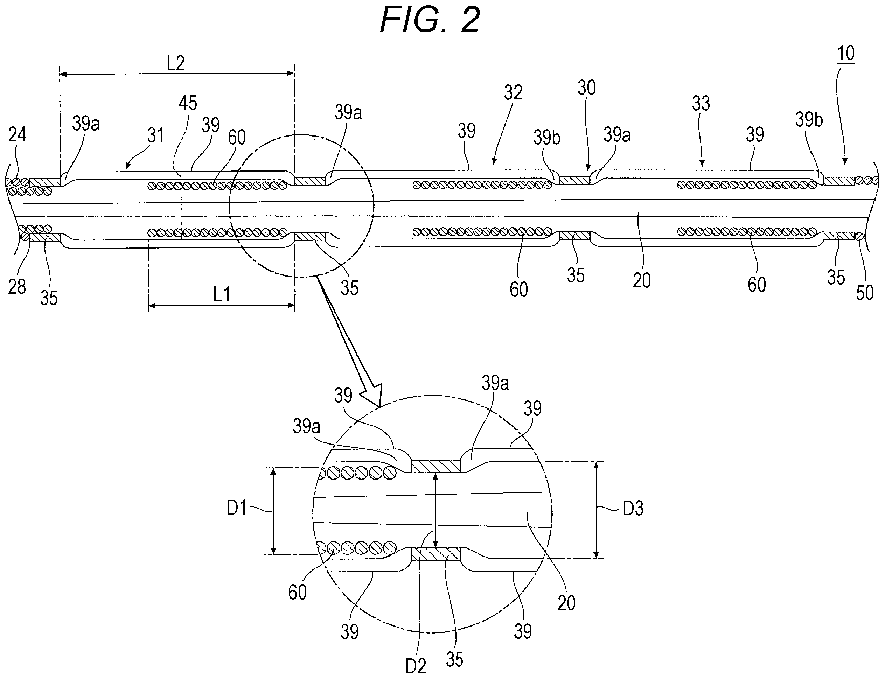

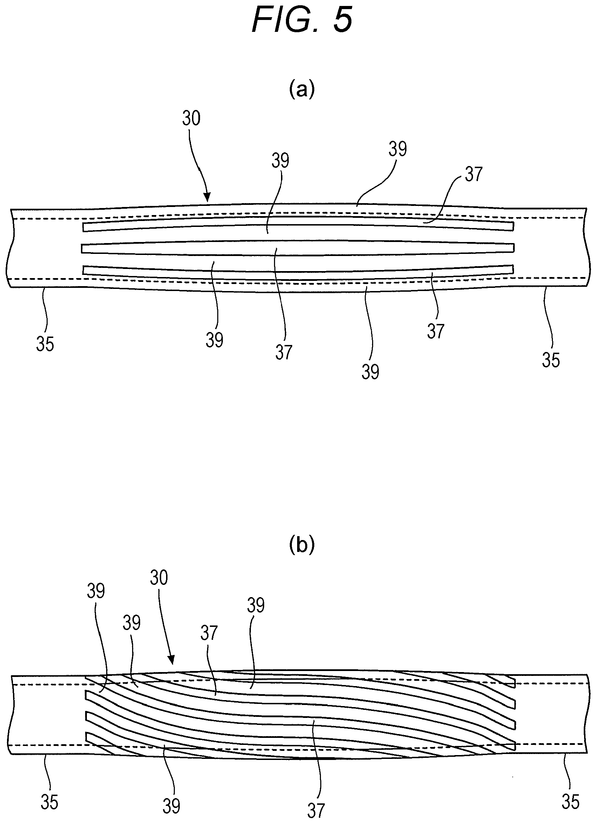

Each of the capturing portions 31, 32, 33 is configured such that, as illustrated in Fig. 5(a), between the plurality of link portions 35, a plurality of slits 37 linearly extending substantially axially is formed circumferentially, and a plurality of deforming portions 39 extending in a band shape is provided with the slits 37 therebetween. Inside the capturing portions 31, 32, 33, the control members 60, which will be described later, are inserted through the slits 37, whereby the deforming portions 39 expand outward, making the link portions 35 be shaped with a reduced diameter relative to the deforming portions 39 (see Fig. 2).

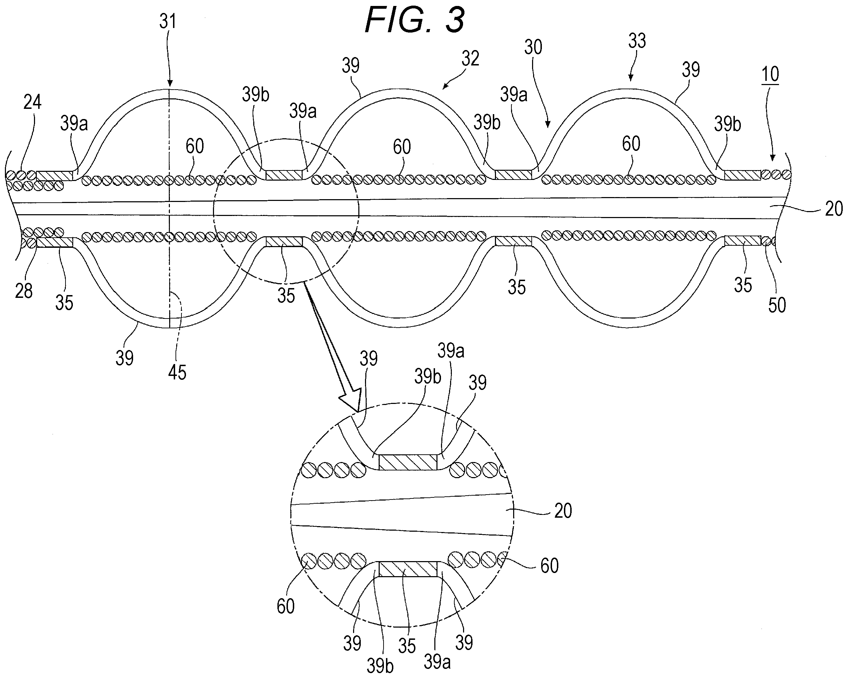

As an axial pressing force acts on the capturing member 30, the axially tip end-side link portion 35 and the axially base end-side link portion 35 become closer to each other, and the deforming portions 39 of the capturing portions 31, 32, 33 contract axially and expand in diameter (see Fig. 3). On the other hand, as the axially tip end-side link portion 35 and the axially base end-side link portion 35 of the capturing member 30 are spaced apart from each other, the deforming portions 39 of the capturing portions 31, 32, 33 extend axially and are reduced in diameter (see Fig. 2).

As illustrated in Fig. 1 and Fig. 2, the control members 60 are respectively disposed inside the capturing portions 31, 32, 33. The control members 60 have an outer diameter greater than an inner diameter of the link portions 35, so that, as noted above, the deforming portions 39 of the capturing portions 31, 32, 33 are expanded outward, making the shape of tip end portions 39a and base end portions 39b, which are portions for linking with the link portions 35, constricted and reduced in diameter. As a result, the control members 60 are unable to pass inside the link portions 35, and are unable to move into the adjacent deforming portions 39. Accordingly, with the movement of the capturing member 30 on the base end side regulated at the tip end portion of the tubular member 50, as the tubular member 50 and the core wire 20 are moved relative to each other and the capturing member 30 is contracted axially via the support member 24, as will be described later, both ends of the control members 60 abut the inner side of both ends 39a, 39b of the deforming portions 39, whereby the amount of axial contraction of each of the capturing portions 31, 32, 33 is regulated, and the deforming portions 39 expands in diameter (see Fig. 3). The diameter expansion operation of the capturing portions 31, 32, 33 will be described in detail with reference to the control members 60 described below.

The capturing member 30 of the present embodiment has three capturing portions 31, 32, 33. However, there may be two or four or more capturing portions, and the number of the capturing portions is not particularly limited. In the capturing member 30 of the present embodiment, a plurality of capturing portions is integrally formed from a single metal tubular member. However, a capturing member including a plurality of capturing portions may be formed by, for example, forming a capturing portion in each of short metal tubular members, and then arranging and fixing them along the axial direction of the core wire 20.

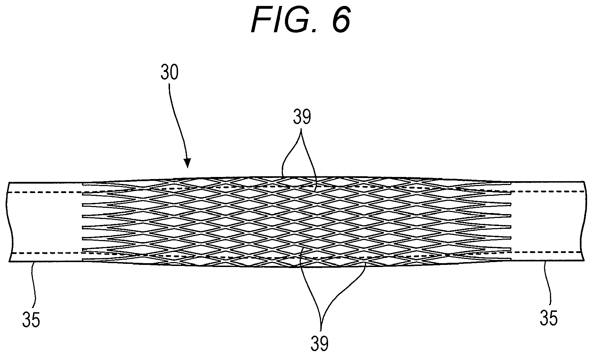

The capturing member of the present embodiment has the structure in which the linearly and substantially axially extending slits 37 are formed, and which has the band-shaped deforming portions 39 (see Fig. 5(a)). However, as illustrated in Fig. 5(b), for example, slits 37 extending substantially spirally with respect to the axial direction of the capturing member may be formed, and a plurality of deforming portions 39 may be provided with the slits 37 therebetween. Further, as illustrated in Fig. 6, meshed deforming portions 39 may be provided. Thus, the shape of the capturing member is not particularly limited as long as the capturing member can contract axially by the length regulated by the control members and expand in diameter.

Further, in the capturing member 30 of the present embodiment, the plurality of capturing portions 31, 32, 33 is formed by the plurality of band-shaped deforming portions 39, formed with the slits 37 therebetween, all extending with the same width. However, this does not represent a limitation.

For example, of the plurality of capturing portions constituting the capturing member, the deforming portions of the capturing portions disposed at both ends in the axial direction of the core wire may be formed thin so as to be narrow in width relative to the deforming portions of the capturing portion disposed at the axial center. In this case, with the movement of the capturing member on the base end side being regulated by the tubular member, as the tubular member and the core wire are moved relative to each other and the capturing member contracts axially, initially the deforming portions of the capturing portions disposed at both ends axially expand in diameter, and then the deforming portions of the capturing portion disposed at the axial center expand in diameter. That is, when capturing a foreign object in a lumen, the capturing portions at both axial ends initially expand in diameter. Accordingly, even if the foreign object, such as a thrombus, becomes scattered by, e.g., being broken, it is possible to stop the scattering by the capturing portions at both axial ends, and to reliably capture the foreign object by the capturing portion at the axial center that later expands in diameter.

Also, of the plurality of capturing portions, the deforming portions of the capturing portion disposed on the tip end side of the core wire may be formed the thinnest, the deforming portions of the capturing portion disposed closer to the base end side of the core wire than said capturing portion may be formed thicker, and the deforming portions of the capturing portion disposed closer to the base end side of the core wire than said capturing portion may be formed even thicker, so that the deforming portions of the capturing portions disposed from the tip end side of the core wire toward the base end side become gradually thicker. For example, the deforming portions 39 of the capturing portion 31 are formed the thinnest, the deforming portions 39 of the capturing portion 32 are formed thicker than that, and the deforming portions 39 of the capturing portion 33 are formed the thinnest. In this case, with the movement of the capturing member on the base end side being regulated by the tubular member, as the tubular member and the core wire are moved relative to each other and the capturing member contracts axially, initially the deforming portions of the capturing portion on the axially tip end side expand in diameter, then the deforming portions of the capturing portion closer to the axially base end side than that expand in diameter, and later the deforming portions of the capturing portion disposed on the axially base end side expand in diameter successively. As a result, for example, when capturing a foreign object in a lumen, even if the foreign object, such as a thrombus, is broken by the expansion in diameter and the like of the deforming portions of the capturing portion on the axially base end side, and tends to scatter upstream in the lumen, because the deforming portions of the capturing portion on the axially tip end side have already expanded in diameter, it is possible to prevent the further upstream scattering of the foreign object in the lumen.

Further, of the plurality of capturing portions 31, 32, 33, the capturing portion 31 disposed at the farthest-tip end is provided with a cover film 45 for capturing a foreign object. In the present embodiment, the cover film 45 is disposed in a range from the tip end to the axial center of the deforming portions 39 of the capturing portion 31, and covers the slits 37 in the range to be able to capture the foreign object so that the foreign object does not goes out via the slits 37. The cover film 45 may be provided so as to cover the deforming portions 39 as a whole of the capturing portion 31. Also, the cover film 45 may not be provided in the capturing portion 31.

As illustrated in Fig. 1, a link member 28 having the shape of a closely wound coil of wire material having a predetermined diameter is disposed outside the core wire 20 between the base end-side inner periphery of the support member 24 and the tip end-side inner periphery of the capturing portion 31. The outer periphery of the link member 28 is fixedly attached to the inner periphery of the support member 24 and to the inner periphery of the tip end-side link portion 35 of the capturing portion 31, using an adhesive or by welding, for example, whereby the farthest tip end of the capturing member 30 is fixed to the tip end portion of the core wire 20 via the support member 24 and is movement-regulated. Thus, with the tubular member 50 being held and fixed, as the core wire 20 is pulled toward the hand, as indicated by an arrow F1 in Fig. 1(a), while the movement of the capturing member 30 is regulated with the tip end portion of the tubular member 50, the farthest tip end of the capturing portion 31 of the capturing member 30 is pressed toward the pull direction side via the support member 24 fixed to the core wire 20. On the other hand, with the core wire 20 being held and fixed, as the tubular member 50 is pressed toward the tip end side relative to the core wire 20, as indicated by an arrow F2 in Fig. 1(a), the tip end portion of the tubular member 50 abuts the base end side of the holding member 30, and the capturing member 30 is pushed from the base end side. However, because at this time the tip end side of the capturing member 30 is supported by the support member 24 fixed to the core wire 20, the farthest tip end of the capturing portion 31 of the capturing member 30 is pressed toward the push-in direction side.

In the present embodiment, the farthest tip end of the capturing member 30 is fixed to the core wire 20. However, the farthest tip end of the capturing member may not be fixed to the core wire 20 as long as its movement is regulated. Also, the link member 28 may not be disposed outside the core wire 20.

Furthermore, the tubular member 50 of the present embodiment has substantially the shape of a cylinder formed of a closely wound coil of wire material having a predetermined diameter. The axial tip end of the tubular member 50 is disposed so as to face the closest base end of the capturing member 30, which herein is the base end of the link portion 35 positioned at the closest base end of the capturing portion 33. The tubular member 50 is not fixed with respect to the core wire 20 and the capturing member 30, and is movable over the outer periphery of the core wire 20. That is, in the present embodiment, the tubular member 50 and the core wire 20 are movable relative to each other. The tubular member may be fixed to the base end side of the capturing member and may be integrally provided with the capturing member.

As described above, in the state in which the tubular member 50 is held and fixed, and as indicated by the arrow F1 in Fig. 1(a), when the core wire 20 is pulled toward the hand relative to the pull tubular member 50 and the farthest tip end of the capturing portion 31 is pressed toward the pull direction side via the support member 24, the base end of the capturing portion 33 abuts the tip end of the tubular member 50 and is supported, whereby the movement of the capturing member 30 on the base end side is regulated. On the other hand, in the state in which the core wire 20 is held and fixed, and as indicated by the arrow F2 in Fig. 1(a), when the tubular member 50 is pressed toward the tip end side relative to the core wire 20, the tip end of the tubular member 50 abuts the base end of the capturing portion 33 of the capturing member 30, whereby the movement of the capturing member 30 on the base end side is regulated. The tubular member may not have the shape of a coil and only needs to have the shape of a tube.

Further, as described above, inside the capturing portions 31, 32, 33, the control members 60 are respectively disposed (see Fig. 1). In addition, as illustrated in Fig. 2, in the present embodiment, the control members 60 are each configured from a coil spring having the shape of a closely wound coil of wire material having a predetermined diameter. Further as illustrated in Fig. 2, the control members 60 have an outer diameter D1 which is greater than an inner diameter D2 of the link portions 35 of the capturing portions 31, 32, 33 of the capturing member 30, and is smaller than an interval D3 of the deforming portions 39, 39 circumferentially disposed in an opposed manner. The control members 60 have an axial length L1 which is smaller than an axial length L2 of the deforming portions 39 of the capturing portions 31, 32, 33, and the control members 60 are axially movable inside each of the capturing portions 31, 32, 33 over the outer periphery of the core wire 20. That is, in the present embodiment, the control members 60 and the core wire 20 are movable relative to each other.

In the present embodiment, the control members 60 are each made of a closely wound coil spring. However, the control members may be coil springs of wire material having a wire-to-wire gap. The control members may not be coil springs and may be instead tube-like bodies or the like having a predetermined length; the control members only need to be able to control the amount of axial contraction of the capturing member. In the present embodiment, the control members 60 do not have both axial ends thereof fixed to the capturing member 30, and the capturing member 30 and the control members 60 are movable relative to each other. However, for example, one axial end of the control members 60 may be fixed to the tip end portions 39a or the base end portions 39b of the deforming portions 39 of the capturing portions 31, 32, 33 of the capturing member 30, and the end of the control members 60 on the side opposite to the fixed end may be movable relative to the capturing member 30.

The foreign object capturing tool 10 is configured such that, by moving the tubular member 50 and the core wire 20 relative to each other, the capturing member 30 contracts axially by the length regulated by the control members 60 and expands in diameter.

For example, with the tubular member 50 being held and fixed, and as indicated by the arrow F1 in Fig. 1(a), as the core wire 20 is pulled toward the hand relative to the tubular member 50, the farthest tip end of the capturing portion 31 is pressed toward the pull direction side via the support member 24, and the movement of the capturing member 30 is regulated by the tubular member 50. As the core wire 20 is further pulled toward the hand from that state, the farthest tip end of the capturing portion 31 is pressed toward the pull direction side via the support member 24, and the deforming portions 39 of the capturing portion 31 expand in diameter. The capturing portion 31 contracts axially and the deforming portions 39 expand in diameter until the tip end of the control member 60 abuts the inner side of the tip end portions 39a of the deforming portions 39 of the capturing portion 31, and until the base end of the control member 60 abuts the inner side of the base end portions 39b of the deforming portions 39. That is, the amount of expansion in diameter of the deforming portions 39 of the capturing portion 31 is controlled by the length of the control members 60. In the capturing portion 32 and the capturing portion 33, the respective deforming portions 39 also contract axially and expand in diameter due to the pulling force from the core wire 20, where the amount of expansion in diameter is regulated by the control members 60.

On the other hand, with the core wire 20 being held and fixed, and as indicated by the arrow F2 in Fig. 1(a), as the tubular member 50 is pressed toward the tip end side relative to the core wire 20, the tip end of the tubular member 50 abuts the base end of the capturing portion 33 of the capturing member 30, and the capturing member 30 is pushed from the base end side. At this time, because the tip end side of the capturing member 30 is supported by the support member 24 fixed to the core wire 20, the farthest tip end of the capturing portion 31 of the capturing member 30 is pressed toward the push-in direction side, and the deforming portions 39 of the capturing portion 31 become expanded in diameter. Then, the capturing portion 31 contracts axially and the deforming portions 39 expand in diameter until the tip end of the control members 60 abuts the inner side of the tip end portions 39a of the deforming portions 39 of the capturing portion 31, and until the base end of the control members 60 abuts the inner side of the base end portions 39b of the deforming portions 39. That is, the amount of expansion in diameter of the deforming portions 39 of the capturing portion 31 is controlled by the length of the control members 60. Similarly, the deforming portions 39 of the capturing portion 32 and the capturing portion 33 also contract axially and expand in diameter due to the push-in force from the tubular member 50, where the amount of expansion in diameter is regulated by the control members 60.

As noted above, in the state in which the movement of the capturing member 30 is regulated by the tubular member 50, by causing the tubular member 50 and the core wire 20 to be moved relative to each other by either pulling the core wire 20 toward the hand relative to the tubular member 50, or pushing the tubular member 50 toward the tip end side relative to the core wire 20, the capturing portions 31, 32, 33 of the capturing member 30 contract axially by the length regulated by the control members 60, and the respective deforming portions 39 expand in diameter. Thus, it is possible to adjust the amount of expansion in diameter of the capturing member 30, that herein is the amount of expansion in diameter of each of the capturing portions 31, 32, 33 (see Fig. 3). The amount of expansion in diameter of the deforming portions 39 of the capturing portions 31, 32, 33 can be adjusted by changing the axial length L1 of the control members 60, as appropriate.

The foreign object capturing tool 10 described above can be manufactured as follows, for example. First, for example, in order to form the capturing portions 31, 32, 33 from a single metal cylindrical member, a plurality of slits 37 is formed at predetermined locations by laser-cutting or the like, thereby forming the band-shaped deforming portions 39. In this way, the capturing member 30 in which the plurality of capturing portions 31, 32, 33 is integrally molded is fabricated. Thereafter, the control members 60 are respectively inserted through the respective slits 37 of the capturing portions 31, 32, 33, and are disposed inside the deforming portions 39. Also, the link member 28 is disposed on the tip-end side of, and externally around, the core wire 20, and the base end inner periphery of the link member 28 is fixedly attached to the inner periphery of the tip end-side link portion 35 of the capturing portion 31.

Then, the core wire 20 is inserted inside the capturing member 30 and the respective control members 60, the support member 24 is disposed on the tip-end side of, and externally around, the core wire 20, and the tip end of the support member 24 and the tip end 21 of the core wire 20 are fixed to each other via the tip end-fixing portion 23. The base end of the support member 24 is fixedly attached to the tip end of the link portion 35 of the capturing portion 31. Further, the tubular member 50 is disposed externally around the base end portion of the core wire 20, the holding member 26 is disposed on the base end side of the tubular member 50, and the base end of the holding member 26 and the base end 22 of the core wire 20 are fixed to each other via the base end-fixing portion 25. As a result, the foreign object capturing tool 10 illustrated in Fig. 1 can be manufactured. The above-described method of manufacturing the foreign object capturing tool 10 is exemplary and does not represent a limitation.

The material of the core wire 20, the capturing member 30, the tubular member 50, and the control members 60 is not particularly limited. Preferable examples include: stainless steel, Ta, Ti, Pt, Au, and W; Ni-Ti-based alloys such as Ni-Ti, Ni-Ti-Co, Ni-Ti-Cu, Ni-Ti-Fe, Ni-Ti-Nb, Ni-Ti-V, Ni-Ti-Cr, and Ni-Ti-Mn; and shape memory alloys including Co-Cr-based alloys such as Co-Cr, Co-Cr-Mo, and Co-Cr-Ni, Cu-Al-based alloys such as Cu-Al-Mn, Cu-Al-Ni, and Cu-Al-Be, and Cu-Zn-based alloys.

Preferably, the control members 60 are made from a radiopaque material including, for example, W, Pt, Ti, Pd, Rh, Au, Ag, Bi, Ta and alloys thereof. When the control members 60 are made from a radiopaque material, as the tubular member 50 and the core wire 20 are moved relative to each other under fluoroscopy, it is possible to monitor changes in density of their images or changes in their intervals. In this case, the control members 60 may be closely wound coils and the like, may have the shape of a coil of wire material having a wire-to-wire gap, or may be ring-shaped members divided into a plurality of sections. Further, when the control members 60 are disposed respectively in the plurality of capturing portions 31, 32, 33 of the capturing member 30, as the tubular member 50 and the core wire 20 are moved relative to each other, the control members 60 in the respective capturing portions become closer to or spaced apart from each other, and it is possible to monitor changes in density of their images or changes in their intervals.

The material of the cover film 45 is not particularly limited. Preferable examples include: polyurethane, silicone, natural rubber, nylon elastomer, polyether block amide, polyethylene, polyvinyl chloride, and vinyl acetate; fluorine resins such as polytetrafluoroethylene (PTFE), perfluoroalkoxy (PFA) resin, tetrafluoroethylene-hexafluoropropylene copolymer (FEP), and tetrafluoroethylene-ethylene copolymer (ETFE); olefin rubbers such as polybutadiene; and styrene elastomer.

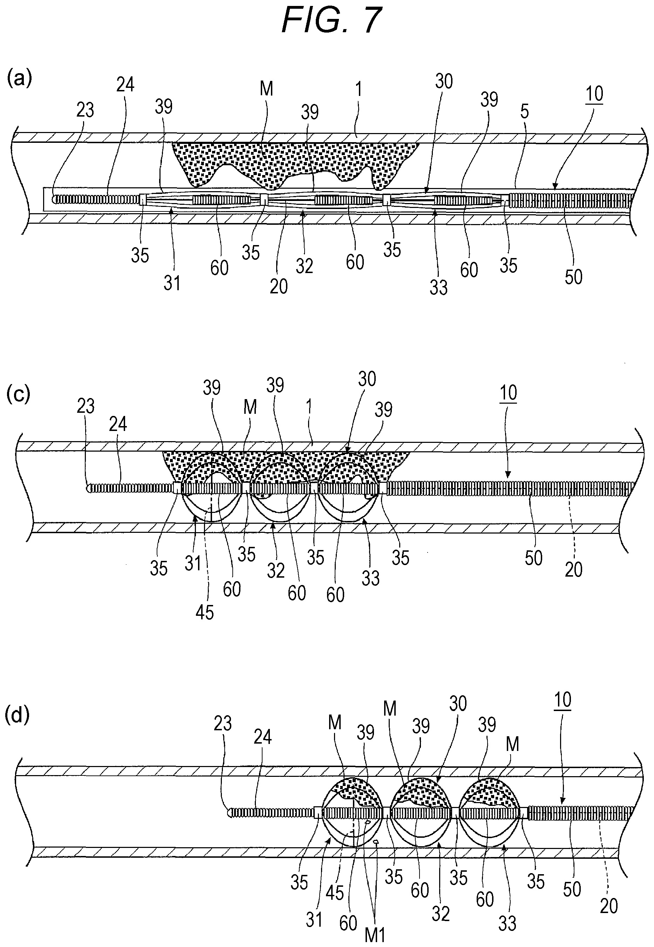

An example of the method of using the foreign object capturing tool 10 having the above-described configuration according to the present invention will be described with reference to Fig. 7. The method of use is an example and does not particularly represent a limitation.

The foreign object capturing tool 10 is used to, for example, capture a foreign object M, such as a gallstone, a pancreatic stone, a ureteral stone, or a thrombus produced in tubular organs, such as a bile duct, a pancreatic duct, a urinary tract, trachea, and blood vessels (including blood vessels in the brain, thoracic aorta, and abdominal aorta) and other lumens, and to eject the foreign object M from within the lumen. The location for applying the foreign object capturing tool 10 is not particularly limited. In the following, a case in which the foreign object M produced in a tubular organ 1 is captured and ejected will be described.

First, as illustrated in Fig. 1(a), with the deforming portions 39 of the capturing portions 31, 32, 33 of the capturing member 30 axially extended, the foreign object capturing tool 10 is accommodated in a catheter 5, as illustrated in Fig. 7(a). Preferably, the catheter 5 has a lumen through which a guide wire can be passed. Then, the catheter 5 is inserted by a known method via a guide wire, which is not shown, until the tip end 21 of the core wire 20 is positioned past a portion in which the foreign object M exists (see Fig. 7(a)).

In this state, the foreign object capturing tool 10 is fixed, the catheter 5 is pulled toward the hand to place the plurality of capturing portions 31, 32, 33 of the capturing member 30 in an exposed state, and then the tubular member 50 and the core wire 20 are moved relative to each other. For example, in a state in which the tubular member 50 is held and fixed, as the core wire 20 is pulled toward the hand relative to the tubular member 50, as indicated by the arrow F1 in Fig. 1(a), the movement of the capturing member 30 is regulated by the tubular member 50, and the farthest tip end of the capturing portion 31 is pressed toward the pull direction side via the support member 24. As a result, the plurality of capturing portions 31, 32, 33 of the capturing member 30 contracts axially by the length regulated by the axial length L1 of the control members 60, and the band-shaped deforming portions 39 expand in diameter. Accordingly, as illustrated in Fig. 7(b), it is possible to cause the deforming portions 39 of the capturing portions 31, 32, 33 to bite into the foreign object M. In this state, the foreign object capturing tool 10 as a whole is pulled toward the hand, whereby the foreign object M can be captured, as illustrated in Fig. 7(c). By further pulling the foreign object capturing tool 10 as a whole toward the hand, it is possible to eject the foreign object M out of the tubular organ 1.

As described above, in the foreign object capturing tool 10, after being inserted into the tubular organ 1 in which the foreign object M exists, the tubular member 50 and the core wire 20 are moved relative to each other by pulling the core wire 20 toward the hand relative to the tubular member 50 or by pushing the core wire 20 toward the tip end side relative to the tubular member 50, whereby the capturing member 30 (herein, the plurality of capturing portions 31, 32, 33) contracts axially by the length regulated by the control members 60 and expands in diameter, producing an expansive force with which it is possible to capture the foreign object M reliably. In addition, because the amount of axial contraction of the capturing member 30 is regulated by the control members 60, it is possible to cause the capturing member 30 to expand to an appropriate diameter to obtain a sufficient expansive force for capturing the foreign object M.

In the present embodiment, the capturing member 30 has the plurality of capturing portions 31, 32, 33 disposed in the axial direction of the core wire 20, and the control members 60 are respectively disposed inside the capturing portions 31, 32, 33. Thus, as the core wire 20 is pulled toward the hand relative to the tubular member 50, as illustrated in Fig. 3 and Fig. 7(b), (c), it is possible to cause each of the plurality of capturing portions 31, 32, 33 to expand in diameter uniformly. In addition, because it is possible to capture the foreign object M by each of the plurality of capturing portions 31, 32, 33, the foreign object M can be captured more reliably, and the amount of the foreign object M that remains unremoved can be reduced.

Further, in the present embodiment, the control members 60 are made of coil springs. Accordingly, the control members 60 can be made flexible, so that, even when the tubular organ 1 or the like in which the foreign object M exists is bent, the foreign object capturing tool 10 can be inserted smoothly, and the amount of expansion in diameter of the capturing member 30 can be adjusted easily. In addition, when the control members 60 are made of coil springs of wire material formed with a wire-to-wire gap, as the capturing member 30 expands in diameter, the coil springs contract against elastic force and the gap between the wires becomes narrower, thereby producing a recovery force. Accordingly, when the capturing member 30 is brought back to the diameter-reduced state, it becomes easier to cause the capturing member 30 to become reduced in diameter.

When the control members 60 is made from a radiopaque material, as the tubular member 50 and the core wire 20 are moved relative to each other under fluoroscopy, it is possible to monitor changes in density of their images or change in their intervals. Accordingly, it is possible to reliably monitor the diameter-expanded state of the capturing member 30, that herein is the diameter-expanded state of the plurality of capturing portions 31, 32, 33.

Further, in the present embodiment, of the plurality of capturing portions 31, 32, 33, the capturing portion 31 disposed at the farthest-tip end is provided with the cover film 45 for capturing the foreign object M. Thus, as illustrated in Fig. 7(c), when the foreign object M is captured by the plurality of capturing portions 31, 32, 33, even if the foreign object M is broken and the like and small fragments of the foreign object M1 tend to scatter, it is possible to capture such small fragments of the foreign object M1 using the cover film 45.

The present invention is not limited to the foregoing embodiment, and various modifications are possible within the spirit and scope of the present invention, and such embodiments are also included in the scope of the present invention.

### LIST OF REFERENCE NUMERALS

- 10: Foreign object capturing tool
- 20: Core wire
- 30: Capturing member
- 31, 32, 33: Capturing portion
- 39: Deforming portion
- 45: Cover film
- 50: Tubular member
- 60: Control member

## Claims

1. An intraluminal foreign object capturing tool for capturing a foreign object in a lumen, comprising:
a core wire;
a capturing member which is disposed movably outside the core wire, of which a farthest tip-end is movement-regulated at a predetermined position of the core wire, and which expands in diameter by contracting axially;
a tubular member which is disposed outside the core wire on a base end side of the capturing member, and which regulates movement of the capturing member on the base end side; and
a control member which is disposed outside the core wire and inside the capturing member, and which regulates an amount of axial contraction of the capturing member,
wherein, as the tubular member and the core wire are moved relative to each other, the capturing member contracts axially by a length regulated by the control member and expands in diameter.

2. The intraluminal foreign object capturing tool according to claim 1, wherein:
the capturing member includes a plurality of capturing portions disposed in an axial direction of the core wire; and
the control member is disposed inside each of the capturing portions.

3. The intraluminal foreign object capturing tool according to claim 2, wherein, of the plurality of the capturing portions, the capturing portion disposed at a farthest-tip end is provided with a cover film for capturing the foreign object.

4. The intraluminal foreign object capturing tool according to any one of claims 1 to 3, wherein the control member is made of a coil spring.

5. The intraluminal foreign object capturing tool according to any one of claims 1 to 4, wherein the control member is made of a radiopaque material, wherein, when the tubular member and the core wire are moved relative to each other under fluoroscopy, it is possible to monitor a change in density of an image thereof or a change in an interval thereof.
